# EUROPÄISCHE PATENTSCHRIFT

(11) **EP 2 581 111 B1**
(45) Veröffentlichungstag und Bekanntmachung des Hinweises auf die Patenterteilung: **04.03.2015**
(21) Anmeldenummer: 13000037.5
(22) Anmeldetag: 13.08.2010
(51) Int. Cl.: A61N 5/10, A61B 19/00

(54) **Vorrichtung zur Ermittlung einer Abweichung zwischen einem durch eine Laseranordnung angezeigten Isozentrum eines Bestrahlungsgeräts und dem tatsächlichen Isozentrum des Bestrahlungsgeräts**
Device for determining a deviation between an isocentre of an irradiation device displayed by a laser assembly and the actual isocentre of an irradiation device
Dispositif de détermination d'une déviation entre un isocentre indiqué par un dispositif à laser d'un appareil de rayonnement et l'isocentre effectif d'un appareil de rayonnement

(43) Veröffentlichungstag der Anmeldung: 17.04.2013
(62) Teilanmeldung aus: 10008463.1
(73) Patentinhaber: LAP GmbH Laser Applikationen, 21337 Lüneburg (DE)
(72) Erfinder: Kindlein, Johann, 21365 Adendorf (DE); Marwedel, Sebastian, 21436 Marschacht (DE); Thurn, Tim, 21335 Lüneburg (DE)
(74) Vertreter: Hauck Patentanwaltspartnerschaft mbB

(56) Entgegenhaltungen:
- EP-A1- 1 854 412
- H TREUER ET AL: "ON ISOCENTRE ADJUSTMENT AND QUALITY CONTROL IN LINEAR ACCELERATOR BASED RADIOSURGERY WITH CIRCULAR COLLIMATORS AND ROOM LASERS", PHYS. MED. BIOL., Bd. 45, 2000, Seiten 2331-2342, XP002608361,

## Beschreibung

Die Erfindung betrifft eine Vorrichtung zur Ermittlung einer Abweichung zwischen einem durch eine Laseranordnung angezeigten Isozentrum eines Bestrahlungsgeräts zur medizinischen Bestrahlung eines Patienten mit hochenergetischer Bestrahlung und dem tatsächlichen Isozentrum des Bestrahlungsgeräts. Die Erfindung betrifft außerdem eine Vorrichtung zur Ermittlung des Isozentrums des Bestrahlungsgeräts und eine Vorrichtung zur Ermittlung einer von einer Laseranordnung angezeigten Position und/oder einer Lage und/oder Ausrichtung von von den Lasern der Laseranordnung ausgesendeten Laserstrahlungsfeldern.

In der Strahlentherapie zur Krebsbehandlung werden Lasermarkierungsvorrichtungen verwendet, um die Lage eines zu behandelnden Tumors erfolgreich an einem Therapiegerät wieder auffinden zu können. Dabei ist es erforderlich räumliche Informationen der diagnostischen Daten auf das Therapiegerät zu übertragen. Es ist ein gebräuchliches Verfahren zur Übertragung der Daten, ein bei der diagnostischen Bildgebung und der anschließenden Bestrahlungsplanung ermitteltes Isozentrum durch eine Projektion von Laserlinien auf einem Patientenkörper anzuzeigen und dort zu markieren. Mithilfe dieser Markierung kann der Patient zu einem späteren Zeitpunkt erneut ausgerichtet werden.

Für die korrekte Übertragung der Daten aus der Bestrahlungsplanung auf die tatsächliche Bestrahlung ist es erforderlich, dass das von den Lasern angezeigte Isozentrum mit dem tatsächlichen Isozentrum des eingesetzten Bestrahlungsgeräts übereinstimmt. Der Task Group 40 Report der Amerikanischen Gesellschaft für Medizinische Physik (AAPM) empfiehlt hierzu eine tägliche Kontrolle der Laserlinienausrichtung. Eine solche Kontrolle ist aufwendig.

In der Praxis kommt es zu Abweichungen zwischen dem von der Laseranordnung angezeigten Isozentrum und dem tatsächlichen Isozentrum des Bestrahlungsgeräts. Gründe hierfür können zum einen Fehljustierungen der Laser sein. Aber auch das tatsächliche Isozentrum eines Bestrahlungsgeräts kann sich verändern. Abweichungen entstehen beispielsweise durch Temperaturveränderungen oder Schwingungen der Wände. Auch kann es zu mechanischen Abweichungen im Zuge der Drehung des Bestrahlungsgeräts kommen ("gantry sag"). Weiterhin kann die besondere Kombination von Bestrahlungsgerät, Patientenunterlage und Kollimatorwinkel zu Abweichungen führen ("couch wobble" und "MLC Offset").

Die Fehlpositionierung eines Patienten bei der Bestrahlung sollte in einem Bereich von weniger als 0,5 mm liegen. Dies erfordert eine hochgenaue Ausrichtung der Markierungslaser und insbesondere eine hochgenaue Abstimmung zwischen dem durch die Markierungslaser angezeigten Isozentrum und dem tatsächlichen Isozentrum des Bestrahlungsgeräts. Heutige Lasersysteme in Bestrahlungsräumen sind fest an einer Wand oder Decke des Raumes befestigt oder werden mit Bodenständern auf dem Fußboden des Raumes installiert. Eine manuelle Feineinstellung der Laser ist mühsam, nicht zuletzt aufgrund der schwierigen Erreichbarkeit. Die Anmelderin hat vor einiger Zeit per Infrarot fernbedienbare Laser unter dem Markennamen "Apollo" auf den Markt gebracht. Mit dem Apollo-System wird die Einstellung von Raumlasern erheblich vereinfacht. Dennoch besteht die Notwendigkeit, in präziser und komfortabler Weise eine Abweichung zwischen einem Isozentrum eines Bestrahlungsgeräts und einem von dem Raumlasersystem angezeigten Isozentrum festzustellen und auszugleichen.

Aus WO 2009/120494 A2 ist ein Dosimeter zur Aufnahme eines Strahlungsbildes eines Linearbeschleunigers bekannt. Das Dosimeter verfügt über ionisierende Detektoren in einer zylindrischen Hülle und erlaubt die Aufnahme des tatsächlichen Strahlungsfeldes. Auf dieser Grundlage kann eine Abweichung zwischen einem Sollstrahlungsfeld und einem tatsächlichen Strahlungsfeld ermittelt werden und ein entsprechender Korrekturwert bestimmt werden. Eine Kalibrierung eines Bestrahlungsgeräts ist auch bekannt aus EP 1 640 922 A2.

Aus Wolfgang W. Baus et al.: "Speicherfoliensystem zur Konstanzprüfung und Dosimetrie am Elektronenlinearbeschleuniger" der Uniklinik Köln ist die Bestimmung des Isozentrum eines Bestrahlungsgeräts mittels eines Speicherfoliensytems auf Grundlage eines sogenannten Sternschusses bekannt. Darüber hinaus ist aus Treuer et al., Phys.Med. Biol. 45 (2000) 2331 bis 2342, ein Verfahren zur Ermittlung des Isozentrums eines Bestrahlungsgeräts bekannt, bei dem zunächst ein für die Strahlung empfindlicher Film bestrahlt wird, beispielsweise mittels eines Sternschusses. Auf dem Film wird mittels einer Nadel das von einem Raumlasersystem angezeigte Isozentrum gekennzeichnet. Auf Grundlage des Filmmaterials wird das Isozentrum des Bestrahlungsgeräts bestimmt, beispielsweise durch Auswerten einer sogenannten kaustischen Kurve. Auf dieser Grundlage kann eine Abweichung zwischen dem von der Raumlaseranordnung angezeigten Isozentrum und dem tatsächlichen Isozentrum des Bestrahlungsgeräts ermittelt werden.

EP 1 854 412 B1 beschreibt darüber hinaus eine Vorrichtung und ein Verfahren zur Überprüfung der Ausrichtung von Laserstrahlen, bei dem ein Trägerelement in definierter Position auf einer Patientenunterlage befestigt wird. Nacheinander werden eine erste und eine zweite Messeinrichtung mit jeweils geeigneten Verbindungsmitteln auf dem Trägerelement befestigt. Die erste Messeinrichtung dient zur Messung der Position der einfallenden Laserstrahlen während die zweite Messeinrichtung die Position des Isozentrums eines Bestrahlungsgeräts aufzeichnet. Die zweite Messeinrichtung besitzt einen Probekörper und ein Aufzeichnungsmaterial, welches zum Aufzeichnen eines Bildes des Probekörpers in dem Behandlungsstrahl ausgebildet ist. Auf diese Weise kann ebenfalls eine Abweichung zwischen dem angezeigten und dem tatsächlichen Isozentrum bestimmt werden.

Die bekannten Vorrichtungen und Verfahren sind allerdings mit einem erheblichen Aufwand verbunden. Dies stellt gerade angesichts der häufigen empfohlenen Kontrolle der Ausrichtung der Raumlaser ein Problem dar. Neben der Ermittlung der Abweichung zwischen den Isozentren betrifft dies insbesondere auch die anschließend erforderliche aufwendige manuelle Neujustierung der Laser.

Es besteht daher ein Bedürfnis nach Vorrichtungen, mit denen in einfacher und komfortabler Weise das Isozentrum eines Bestrahlungsgeräts und das von einem Raumlasersystem angezeigte Isozentrum ermittelt und miteinander verglichen werden können. Auch besteht ein Bedürfnis dahingehend, die Ausrichtung von Raumlasersystemen schnell und präzise überprüfen zu können.

Der Erfindung liegt die Aufgabe zugrunde, Vorrichtungen der eingangs genannten Art bereitzustellen, mit denen die Ermittlung des Isozentrum eines Bestrahlungsgeräts beziehungsweise des von einer Laseranordnung angezeigten Isozentrums und der Abgleich zwischen diesen Positionen in einfacher, komfortabler und präziser Weise möglich ist.

Die Erfindung löst diese Aufgabe durch den Gegenstand von Anspruch 1. Vorteilhafte Ausgestaltungen finden sich in den abhängigen Ansprüchen, der Beschreibung und den Figuren.

Ein Beispiel betrifft eine Vorrichtung zur Ermittlung des Isozentrums eines Bestrahlungsgeräts zur medizinischen Bestrahlung eines Patienten mit hochenergetischer Strahlung, umfassend eine Strahlendetektoreinrichtung mit einer Detektorscheibe, auf der mindestens ein Strahlendetektor zum Detektieren von von dem Bestrahlungsgerät ausgesendeter hochenergetischer Strahlung angeordnet ist, wobei ein Rotationsantrieb vorgesehen ist, mit dem die Detektorscheibe um ihre Zentralachse drehbar ist, und umfassend eine Auswerteeinrichtung, die dazu ausgebildet ist, aus den von dem mindestens einen Strahlendetektor detektierten Messwerten das Isozentrum des Bestrahlungsgeräts zu bestimmen.

Bei dem Bestrahlungsgerät kann es sich insbesondere um einen Linearbeschleuniger (LINAC) handeln, der ionisierende Strahlung zur Behandlung eines Krebstumors erzeugt. Das Isozentrum eines solchen Bestrahlungsgeräts ist üblicherweise definiert als der Schnittpunkt der Rotationsachse des Bestrahlungsgeräts mit dem senkrecht zu dieser Rotationsachse verlaufenden Zentralstrahl des Bestrahlungsgeräts. Die Detektorscheibe der erfindungsgemäßen Vorrichtung liegt zur Messung in der Ebene, in der sich die Zentralstrahlen des Bestrahlungsgeräts bei einer Rotation desselben um die hierfür vorgesehene Rotationsachse bei einer ordnungsgemäßen Positionierung befinden müssten. Die Detektorscheibe liegt also in der Ebene, in der sich der Zentralstrahl des Bestrahlungsgeräts dreht. Die Zentralachse beziehungsweise Rotationsachse der Detektorscheibe verläuft in dieser Stellung parallel zu der Längsachse einer Patientenunterlage. Die Detektorscheibe kann transparent für Laserstrahlung und/oder Strahlung des Bestrahlungsgeräts sein, um eine Messung solcher Strahlung nicht zu stören.

Zur Ermittlung des Isozentrums kann beispielsweise in regelmäßigen Winkelabständen während der Drehung des Bestrahlungsgeräts ein Aussenden eines Zentralstrahls erfolgen. Es kann ein sogenannter Sternschuss vorgenommen werden. Wenn das Isozentrum des Bestrahlungsgeräts keinen mechanischen oder anderen Störungen unterliegt, sollten sich die Zentralstrahlen dann in einem Punkt, nämlich dem Isozentrum schneiden. In der Praxis ist dies aus den oben genannten Gründen allerdings nicht immer der Fall. Dann muss das Isozentrum anhand der Messaufnahmen der Zentralstrahlen angenährt werden. Dies erfolgt durch die Auswerteeinrichtung in an sich bekannter Weise, wie dies beispielhaft im oben erläuterten Stand der Technik beschrieben ist.

Die Detektorscheibe wird während des Aussendens der Strahlung durch das Bestrahlungsgerät ebenfalls rotiert. Aufgrund der Rotation und anhand der Zeitpunkte, zu denen einer oder mehrere Messdetektoren auf der Detektorscheibe Strahlung von dem Bestrahlungsgerät erfassen, kann beispielsweise eine unerwünschte Verschiebung des Zentralstrahls in Richtung der Rotationsachse des Bestrahlungsgeräts und/oder eine unerwünschte Verkippung des Zentralstrahls festgestellt werden. Die Drehung der Detektorscheibe erlaubt dabei in einfacher und komfortabler Weise eine präzise Erkennung solcher unerwünschter Abweichungen, seien diese durch Temperaturänderungen, Gebäudeschwingungen oder mechanische Gründe verursacht.

Es kann weiterhin ein Translationsantrieb vorgesehen sein, mit dem die Detektorscheibe entlang ihrer Zentralachse verschiebbar ist. Gemäß dieser Ausgestaltung ist die Detektorscheibe also entlang ihrer Rotationsachse und somit senkrecht zu ihrer Scheibenoberfläche verschiebbar und gleichzeitig oder nacheinander um ihre Rotationsachse drehbar. Durch die Translationsbewegung kann ein bestimmtes Messvolumen auch in Richtung der Rotationsachse des Bestrahlungsgeräts abgedeckt werden. Es ist dann möglich, eine Verschiebung oder Verkippung des Zentralstrahls des Bestrahlungsgeräts über einen größeren Bereich und damit präziser zu erfassen. Auch ist es bei dieser Ausgestaltung möglich, die Detektorscheibe vor und/oder nach einer Messung vollständig aus dem Messbereich herauszufahren, um beispielsweise eine andere Messung nicht zu stören.

Die Messauflösung wird verbessert, wenn auf mindestens einer Scheibenfläche der Detektorscheibe eine Mehrzahl von Strahlendetektoren angeordnet ist. Als besonders vorteilhaft hat sich eine S-förmige Anordnung der Strahlendetektoren auf der Scheibenfläche herausgestellt. Die beiden Arme der S-Form können jeweils einen Radius besitzen, der doppelt so groß ist wie der Radius der Scheibe. Durch eine solche Sensoranordnung kann der Zentralstrahl des Bestrahlungsgeräts besonders sicher und vollständig erfasst werden. Insbesondere bei einer Sternschussmessung kann eine Auswertung der aufgenommenen Messdaten in an sich bekannter und zu dem oben erläuterten Stand der Technik beschriebener Weise erfolgen. Ebenso können durch die Auswerteeinrichtung das zeitliche Auftreten von Messergebnissen an den einzelnen Strahlendetektoren ausgewertet und daraus Rückschlüsse auf den Verlauf der Strahlung des Bestrahlungsgeräts gezogen werden.

Nach einer weiteren Ausgestaltung kann vorgesehen sein, dass die Vorrichtung ein Bestrahlungsgerät zur medizinischen Bestrahlung eines Patienten mit hochenergetischer Strahlung und eine Strahlungsdetektorsteuereinrichtung umfasst, wobei die Strahlungsdetektorsteuereinrichtung dazu ausgebildet ist, das Bestrahlungsgerät so anzusteuern, dass es um seine Rotationsachse gedreht wird und während der Rotation von dem Bestrahlungsgerät mehrfach ein Zentralstrahl ausgesandt wird, und wobei die Strahlungsdetektorsteuereinrichtung weiter dazu ausgebildet ist, während des Aussendens des Zentralstrahls die Detektorscheibe der Strahlendetektoreinrichtung um ihre Zentralachse zu drehen und den mindestens einen Strahlendetektor so anzusteuern, dass dieser die von dem Bestrahlungsgerät ausgesandten Zentralstrahlen detektiert.

Die Erfindung löst die Aufgabe durch eine Vorrichtung zur Ermittlung einer von einer Laseranordnung mit mehreren Lasern angezeigten Position und/oder einer Lage und/oder Ausrichtung von von den Lasern ausgesendeten Laserstrahlungsfeldern, umfassend eine Laserdetektoreinrichtung mit einer Detektorscheibe, auf der mindestens ein Laserdetektor mit einem sich zumindest in einer Richtung senkrecht zur Scheibenoberfläche der Detektorscheibe erstreckenden Detektorfeld zum Detektieren von von der Laseranordnung ausgesendeter Laserstrahlung, wobei ein erster Rotationsantrieb vorgesehen ist, mit dem die Detektorscheibe um ihre Zentralachse drehbar ist, und wobei ein zweiter Rotationsantrieb vorgesehen ist, mit dem der mindestens eine Laserdetektor um eine parallel zur Zentralachse der Detektorscheibe verlaufende Drehachse drehbar ist, und umfassend eine Auswerteeinrichtung, die dazu ausgebildet ist, aus den von dem mindestens einen Laserdetektor detektierten Messwerten die von der Laseranordnung angezeigte Position und/oder die Lage und/oder Ausrichtung von von den Lasern ausgesendeten Laserstrahlungsfeldern zu bestimmen. Die von der Laseranordnung angezeigte Position kann die Position des Isozentrums eines Bestrahlungsgeräts sein.

Die Laseranordnung kann beispielsweise fünf Laser umfassen. Eine solche Anordnung ist zum Beispiel bekannt aus dem Apollo-System der Anmelderin oder der Druckschrift EP 0 687 443 B1, die ebenfalls auf die Anmelderin zurückgeht. Das Anzeigen beziehungsweise die Markierung einer vorbestimmten Position durch die Laseranordnung kann erfolgen, indem sich die Laserstrahlen an der zu markierenden Position (beispielsweise auf einem auf einer gegebenenfalls verfahrbaren Unterlage liegenden Patientenkörper) in einem Kreuz schneiden. Die einzelnen Laser können jeweils eine in einer Ebene liegende fächerartige Laserstrahlungsfläche erzeugen, sodass sie jeweils eine Linie auf einen Patientenkörper projizieren. Sofern kein Patient auf der Unterlage liegt, sollten sich bei ordnungsgemäßer Justage alle Laserstrahlungsfelder in einem Punkt schneiden. Bei diesem Punkt kann es sich beispielsweise um das Isozentrum des Bestrahlungsgeräts handeln, welches auf diese Weise von der Laseranordnung angezeigt wird.

Erfindungsgemäß werden die Laser so angesteuert, dass sie die vorbestimmte Position, beispielsweise das Isozentrum des Bestrahlungsgeräts, anzeigen. Für die erfindungsgemäße Kalibrierung befindet sich auf der Patientenunterlage das Messphantom mit der Laserdetektoreinrichtung. Dieses Messphantom nimmt mit der Laserdetektoreinrichtung die Laserstrahlungsfelder der einzelnen Laser nacheinander auf. Hierzu wird die Detektorscheibe mit ihrer Scheibenoberfläche parallel zu der Ebene der Oberfläche einer Patientenunterlage angeordnet. Die Drehung der Detektorscheibe erfolgt somit parallel zu dieser, in der Regel waagerechten Ebene. Der mindestens eine Laserdetektor steht in dieser Position nach oben (und/oder nach unten) von der Detektorscheibe vor. Zur Messung können der oder die Laserdetektoren so angeordnet werden, dass die Lasermarkierungen bei ordnungsgemäßer Justage von diesem in vorbestimmter Weise aufgenommen werden, beispielsweise zentral auf einem Detektorfeld. Besteht eine Abweichung zu der tatsächlichen vorbestimmten Position, also beispielsweise dem tatsächlichen Isozentrum, wird dies mittels der optischen Detektoren und der Auswerteeinrichtung festgestellt. Auch kann mit zumindest in einer Richtung ausgedehnten Laserdetektoren die Lage und Orientierung (z.B. die Neigung) der von den Lasern jeweils erzeugten Laserstrahlungsflächen überprüft werden. Auf dieselbe Weise kann auch eine Koplanarität unterschiedlicher Markierungslaser, beispielsweise gegenüberliegender Laser, überprüft werden.

Wie schon bei der Strahlendetektorvorrichtung ist die Grundidee auch bei der erfindungsgemäßen Laserdetektorvorrichtung die drehbare Anordnung der Detektorscheibe, wobei auf dieser mindestens ein Laserdetektor angeordnet ist. Auf diese Weise kann wiederum eine Messebene, beziehungsweise bei einer Ausdehnung des Laserdetektors in mindestens einer Richtung ein Messvolumen abgedeckt werden. Auf diese Weise können die jeweils in eine Ebene strahlenden Raumlaser umfassend vermessen und mittels der Auswerteeinrichtung ausgewertet werden.

Durch die zusätzliche Drehbarkeit des Laserdetektors gegenüber der Detektorscheibe kann sichergestellt werden, dass dieser auch während der Drehung der Detektorscheibe jederzeit dem jeweils zu vermessenden Laser zugewandt ist. Entsprechend kann gemäß einer weiteren Ausgestaltung vorgesehen sein, dass eine Laserdetektorsteuereinrichtung vorgesehen ist, die dazu ausgebildet ist, für zumindest einige der Laser, insbesondere für alle Laser, der Laseranordnung nacheinander jeweils die ersten und zweiten Rotationsantriebe so anzusteuern, dass die Drehung der Detektorscheibe und die Drehung des Laserdetektors derart aufeinander abgestimmt ist, dass das Detektorfeld des Laserdetektors während der Drehung der Detektorscheibe jederzeit der Strahlungsquelle jeweils eines Lasers zugewandt ist, und dass die Laserdetektorsteuereinrichtung dazu ausgebildet ist, den mindestens einen Laserdetektor während der Drehung so anzusteuern, dass dieser auf das Detektorfeld auftreffende Laserstrahlung detektiert. Bei dieser Ausgestaltung steuert die Steuereinrichtung den Rotationsantrieb derart an, dass sich zum einen die Detektorscheibe dreht und dabei das von dem jeweils zu vermessenden Laser ausgesendete Strahlungsfeld insbesondere vollständig überstreicht. Gleichzeitig steuert die Steuereinrichtung den zweiten Rotationsantrieb so an, dass das Detektorfeld des Detektors jederzeit auf den zu vermessenden Laser gerichtet ist. Wie bereits erwähnt, strahlen die Laser in der Regel einen in einer Ebene liegenden Strahlungsfächer aus. Dieser deckt einen bestimmten Winkelbereich ab, sodass auf einen Patientenkörper eine Linie projiziert wird. Es kann somit eine zentrale Ausbreitungsrichtung bei der Hälfte des Winkelbereichs definiert werden. Die Detektor fläche kann dann immer in einer Ebene liegen, die senkrecht zu dieser zentralen Ausbreitungsrichtung liegt.

Es wird darauf hingewiesen, dass auch ein gemeinsamer Rotationsantrieb für die Detektorscheibe und den Laserdetektor möglich ist. Insbesondere ist eine beispielsweise mechanische Kopplung der Drehbewegungen denkbar derart, dass der Laserdetektor bei einer Drehung der Detektorscheibe automatisch abgestimmt ebenfalls gedreht wird. Es müsste in einem solchen Fall nur die Detektorscheibe drehend angetrieben werden. Der zweite Rotationsantrieb wäre dann durch die Kopplung der Drehbewegungen, beispielsweise ein entsprechendes Getriebe, gebildet.

Nach einer weiteren Ausgestaltung kann das Detektorfeld einen sich in einer Richtung senkrecht zur Scheibenfläche der Detektorscheibe erstreckenden Zeilensensor aufweisen. Es handelt sich dabei um einen linearen, optischen Detektor, beispielsweise ein eindimensionales CMOS- oder CCD-Array. Mit solchen Sensoren kann die Position und Neigung der Laserflächen sowohl von seitlich als auch von oberhalb der Patientenunterlage angeordneten Lasern erfasst werden.

Nach einer weiteren Ausgestaltung kann die Detektorscheibe eine durch den ersten Rotationsantrieb um einen feststehenden zentralen Abschnitt drehbare Ringscheibe aufweisen, wobei der zentrale Abschnitt eine Ausnehmung besitzt.

Bei beiden oben beschriebenen erfindungsgemäßen Vorrichtungen können die Detektorscheiben insbesondere eine zylindrische Form besitzen. Es sind aber auch andere Formen für die Detektorscheibe denkbar.

Ein weiteres Beispiel betrifft eine Vorrichtung zur Ermittlung einer Abweichung zwischen einem durch eine Laseranordnung angezeigten Isozentrum eines Bestrahlungsgeräts und dem tatsächlichen Isozentrum des Bestrahlungsgeräts, umfassend
- eine Laseranordnung mit mehreren Lasern und eine Lasersteuereinrichtung, die dazu ausgebildet ist, die Laser der Laseranordnung so anzusteuern, dass diese das Isozentrum anzeigen und eine Laserdetektoreinrichtung, die dazu ausgebildet ist, von den Lasern ausgesendete Laserstrahlung zu detektieren,
- eine erfindungsgemäße Vorrichtung zur Ermittlung des Isozentrums eines Bestrahlungsgeräts, und
   eine Auswerteeinrichtung, die dazu ausgebildet ist, aus von der Laserdetektoreinrichtung und der Strahlendetektoreinrichtung aufgenommenen Messdaten eine Abweichung zwischen dem durch die Laseranordnung angezeigten Isozentrum und dem tatsächlichen Isozentrum zu ermitteln.

Wiederum werden die Laser mit dieser Vorrichtung so angesteuert, dass sie das Isozentrum anzeigen. Bei dieser Ausgestaltung befindet sich im Messbereich ein kombiniertes Messphantom mit einer Vorrichtung zur Bestimmung des tatsächlichen Isozentrum des Bestrahlungsgerätes und einem intelligenten Raumlasersystem, mit welchem sich in einfacher Weise zumindest eine von den Raumlasern angezeigte Position ermitteln lässt. Weiterhin ist eine Auswerteeinrichtung vorgesehen, die eine Abweichung zwischen den ermittelten Positionen bestimmt und gegebenenfalls anzeigt. Auf dieser Grundlage können die Raumlaser in einfacher Weise neu justiert werden, sodass sie das tatsächliche Isozentrum des Bestrahlungsgeräts korrekt anzeigen.

Die Steuereinrichtung kann weiterhin dazu ausgebildet sein, auf Grundlage der von der Auswerteeinrichtung ermittelten Positionsabweichungen die Laser automatisch so anzusteuern, dass das von ihnen angezeigte Isozentrum dem tatsächlichen Isozentrum entspricht. Mit dieser Vorrichtung können also Abweichungen zwischen dem tatsächlichen Isozentrum und dem von den Laserlinien angezeigten Isozentrum durch dasselbe Computersystem automatisch ermittelt werden, in dem entsprechende Messdaten ausgewertet werden. Eventuelle Positionsabweichungen können durch die Steuereinrichtung dann vollautomatisch korrigiert werden. Dem Anwender wird auf diese Weise eine schwierige und zeitaufwendige manuelle Einstellung erspart. Auch eine tägliche Kontrolle der Einstellung ist mit diesem intelligenten Raumlasersystem in kurzer Zeit problemlos möglich.

Gemäß einer Weiterbildung ist es selbstverständlich auch möglich, auf Grundlage der ermittelten Abweichung zwischen tatsächlichem Isozentrum und angezeigtem Isozentrum eine Patientenunterlage und somit auch einen auf der Unterlage befindlichen Patienten neu zu positionieren. All dies kann mit den erfindungsgemäßen Auswerte- und Steuereinrichtungen in automatischer Weise erfolgen.

Die Software zur Auswertung der Messdaten und zur entsprechenden Steuerung kann auf dem ohnehin in dem Bestrahlungsraum vorhandenen Computersystem installiert sein, sodass sämtliche Vorgänge mit demselben Computersystem durchgeführt werden können. Mit der Laserdetektionskomponente des erfindungsgemäßen Messphantoms ist in automatischer Weise eine Kontrolle der Ausrichtung der Raumlaser zur Ermittlung der kritischen Parameter möglich, insbesondere des Schnittpunkts der Laserlinien, die Koplanarität der Laserlinien sowie der Lage der Laserflächen, insbesondere einer Orthogonalität der Laserlinien. Das Raumlasersystem kann auf dieser Grundlage und unter Berücksichtigung des bestimmten Isozentrums des Bestrahlungsgeräts die erforderliche Neujustierung automatisch vornehmen.

Sämtliche oder einige der Auswerteeinrichtungen bzw. der Steuereinrichtungen der erfindungsgemäßen Vorrichtung können als separate oder jeweils gemeinsame Auswerteeinrichtung bzw. Steuereinrichtung ausgebildet sein. Auch können einige oder sämtliche der Auswerteeinrichtungen und Steuereinrichtungen als gemeinsame Auswerte- und Steuereinrichtung(en) ausgebildet sein.

Nach einer weiteren Ausgestaltung kann die Detektorscheibe der Strahlendetektoreinrichtung derart in der Ausnehmung des zentralen Abschnitts der Detektorscheibe der Laserdetektoreinrichtung angeordnet sein, dass die Drehachsen der Detektorscheiben der Strahlendetektoreinrichtung und der Laserdetektoreinrichtung senkrecht zueinander stehen. Auf diese Weise ist eine besonders einfache Kombination der Laserdetektoren und der Strahlendetektoren in einer kompakten Vorrichtung möglich. Die Ausnehmung ist also groß genug, um die Detektorscheibe des Strahlendetektors aufzunehmen.

Die Laserdetektoreinrichtung kann nach einer weiteren Ausgestaltung mindestens eine Kamera umfassen. Es können insbesondere drei Kameras vorgesehen sein, sodass auch fünf Laser, wie beispielsweise bei dem Apollo-System der Anmelderin, vermessen werden können. Es kann damit auch die Koplanarität verschiedener Laserstrahlebenen überprüft werden. Weiterhin kann vorgesehen sein, dass die Laserdetektoreinrichtung mindestens einen Matrixdetektor umfasst. Dem mindestens einen Matrixdetektor kann jeweils mindestens ein Prisma optisch vorgeschaltet sein. Es können wiederum insbesondere drei Matrixdetektoren vorgesehen sein. Bei den Matrixdetektoren kann es sich beispielsweise um zweidimensionale Sensor-Arrays, beispielsweise CMOS- oder CCD-Arrays handeln.

Die erfindungsgemäßen Steuereinrichtungen sind insbesondere dazu ausgebildet, die jeweils beschriebenen Funktionsweisen der Vorrichtungen durchzuführen.

Ein Ausführungsbeispiel der Erfindung wird nachfolgend anhand von Figuren näher erläutert. Es zeigen schematisch:
- Figur 1: eine Vorrichtung gemäß einem ersten Beispiel,
- Figur 2: ein Detail der in Figur 1 dargestellten Vorrichtung, und
- Figur 3: eine erfindungsgemäße Vorrichtung.

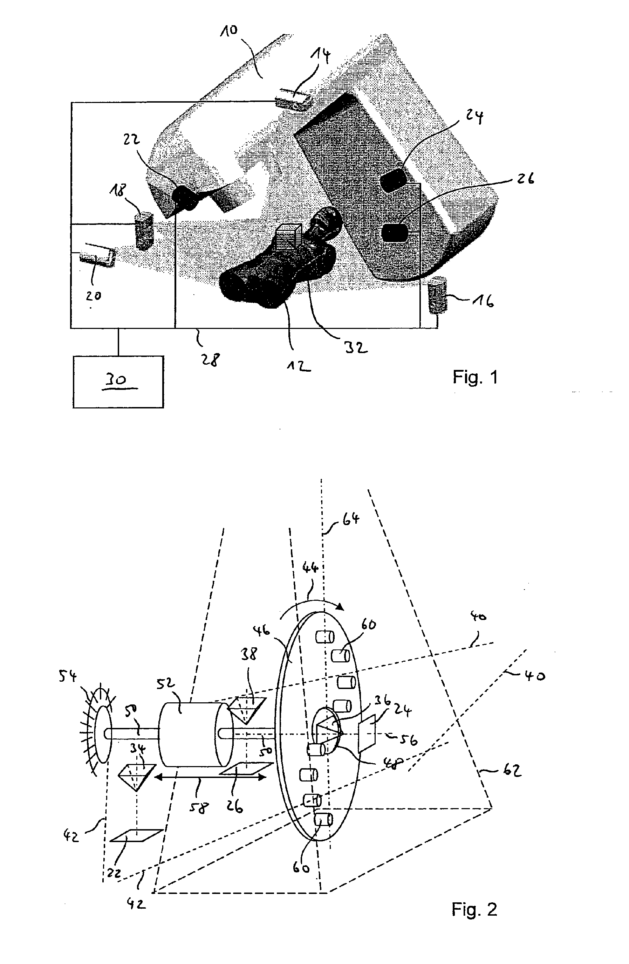

Soweit nichts anderes angegeben ist, bezeichnen in den Figuren gleiche Bezugszeichen gleiche Gegenstände. Die in Figur 1 dargestellte Vorrichtung weist ein Bestrahlungsgerät 10 zur medizinischen Bestrahlung eines bei dem Bezugszeichen 12 schematisch dargestellten Patienten mit ionisierender Strahlung auf. Bei dem Bestrahlungsgerät handelt es sich um einen an sich bekannten Linearbeschleuniger (LINAC), der um eine Rotationsachse drehbar ist. Die Vorrichtung weist darüber hinaus ein Raumlasersystem mit im dargestellten Beispiel vier Lasern 14, 16, 18, 20 auf. Die Laser 14, 16, 18, 20 erzeugen jeweils ein fächerartiges Strahlungsfeld, welche auf den Patientenkörper jeweils eine Linie projizieren. Die beiden Laser 16, 18 sind seitlich des Patienten angeordnet und projizieren eine im Wesentlichen waagerechte Linie auf den Patientenkörper. Der Laser 14 ist oberhalb des Patienten angeordnet und projiziert eine Transversallinie auf den Patientenkörper. Der Laser 20 ist ebenfalls oberhalb des Patientenkörpers 12 angeordnet und projiziert eine Sagitallinie auf den Patientenkörper. Die Vorrichtung umfasst in dem dargestellten Beispiel darüber hinaus drei optische Detektoren 22, 24, 26. Über Leitungen 28 sind die Laser und die optischen Detektoren mit einer Auswerte- und Steuereinrichtung 30 verbunden. An der Position des Patientenkörpers 12 ist in Figur 1 höchst schematisch ein Messphantom 32 dargestellt. Mit diesem Messphantom 32 in Verbindung mit den optischen Detektoren 22, 24, 26 kann zum einen das Isozentrum des Bestrahlungsgeräts 10 bestimmt werden und zum anderen beispielsweise ein von den Lasern der Laseranordnung angezeigtes Isozentrum ermittelt werden. Mittels der Auswerte- und Steuereinrichtung 30 kann eine Abweichung zwischen diesen Isozentren ermittelt und eine automatische Neujustierung der Laser der Laseranordnung durchgeführt werden. Dies soll anhand des in Figur 2 dargestellten Details näher erläutert werden.

In Figur 2 sind die Laser 14, 16, 18, 20 sowie das Bestrahlungsgerät 10 und die Auswerte- und Steuereinrichtung 30 aus Gründen der Übersichtlichkeit nicht dargestellt. Die in Figur 1 höchst schematisch dargestellten optischen Detektoren 22, 24, 26 sind in Figur 2 dagegen gezeigt. In dem dargestellten Ausführungsbeispiel handelt es sich um zweidimensionale Matrixdetektoren. Ihnen ist jeweils ein Prisma 34, 36, 38 optisch vorgeschaltet. Mit den Matrixdetektoren 22, 24, 26 und den ihnen vorgeschalteten Prismen 34, 36, 38 können die von den Lasern erzeugten Laserstrahlungsfelder, von denen in Figur 2 lediglich zwei bei den Bezugszeichen 40, 42 angedeutet sind, erfasst und vermessen werden. Insbesondere kann durch Auswerten der Messwerte der Matrixkameras 22, 24, 26 eine Position der Laserlinien sowie eine Lage und Neigung und damit auch Koplanarität und Orthogonalität der entsprechenden Laserebenen ermittelt werden. Dies erfolgt mittels der kombinierten Auswerte- und Steuereinrichtung 30.

Darüber hinaus ist in Figur 2 im Detail die Ausgestaltung des in Figur 1 höchst schematisch dargestellten Messphantoms 32 gezeigt. Dieses Messphantom 32 weist eine mittels eines Drehantriebs, vorliegend eines Motors 52, wie schematisch bei dem Pfeil 44 gezeigt, rotierend antreibbare zylindrische Detektorscheibe 46 auf. Die Detektorscheibe 46 besitzt eine zentrale Ausnehmung 48 und ist über eine Welle 50 mit dem Motor 52 verbunden. Ebenfalls mit der Welle 50 verbunden ist ein Winkelgeber 54. Weiterhin ist ein Translationsantrieb (nicht dargestellt) vorgesehen, mit dem eine Translation der Detektorscheibe 46 entlang der Rotationsachse 56 möglich ist, wie durch den Pfeil 58 veranschaulicht. S-förmig auf der Scheibenoberfläche der Detektorscheibe 46 verteilt sind mehrere Strahlungsdetektoren 60 angeordnet. Bei den Strahlungsdetektoren 60 handelt es sich um ionisierende Detektoren, mit denen von dem Bestrahlungsgerät 10 ausgesandte ionisierende Strahlung gemessen werden kann. Bei dem Bezugszeichen 62 in Figur 2 ist das Bestrahlungsfeld des Bestrahlungsgeräts 10 in einer Drehposition angedeutet. Bei dem Bezugszeichen 64 ist der Zentralstrahl des Bestrahlungsgeräts 10 in einer Drehposition gezeigt. In dieser Drehposition verläuft der Zentralstrahl 64 in vertikaler Richtung.

Mit dem derart aufgebauten Messphantom 32 ist eine Bestimmung des Isozentrums des Bestrahlungsgeräts 10 möglich. Dazu wird ebenfalls von der in Figur 1 dargestellten Auswerte- und Steuereinrichtung 30 die Detektorscheibe 46 mittels des Translationsantriebs für einen Messvorgang entlang des Pfeils 58 in die Messebene verfahren, in der sich der Zentralstrahl 64 des Bestrahlungsgeräts 10 bei ordnungsgemäßer Einstellung drehen sollte. In dieser Ebene wird die Detektorscheibe 46 von der Auswerte- und Steuereinrichtung 30 in Rotation versetzt. Während der Rotation wird von dem Bestrahlungsgerät 10 in unterschiedlichen Drehpositionen ein Zentralstrahl 64 ausgesandt und von den Strahlungsdetektoren 60 gemessen. Anhand der von den einzelnen Detektoren 60 erfassten Strahlungsintensität sowie der Zeitpunkte der Strahlungsdetektion kann die Auswerte- und Steuereinrichtung 30 auf die Lage und Neigung der Zentralstrahlen 64 in den unterschiedlichen Drehpositionen des Bestrahlungsgeräts 10 schließen. Auch ein Schnittpunkt der Zentralstrahlen kann ermittelt bzw. angenähert werden. Um die Messgenauigkeit in dieser Hinsicht zu erhöhen, wird der Translationsantrieb von der Auswerte- und Steuereinrichtung 30 während der Vermessung jedes Zentralstrahls 64 derart angesteuert, dass die Detektorscheibe 46 einen bestimmten Translationsbereich überstreicht. Auf diese Weise kann eine Verkippung des Zentralstrahls 64 vollständiger erfasst werden. Auf Grundlage der Messergebnisse der Strahlendetektoren 60 einerseits und der optischen Detektoren 22, 24, 26 andererseits kann eine Abweichung zwischen dem tatsächlichen Isozentrum des Bestrahlungsgeräts 10 und dem von der Laseranordnung angezeigten Isozentrum festgestellt und durch eine mittels der Auswerte- und Steuereinrichtung 30 bewirkte automatische Neujustierung der Laser 14, 16, 18, 20 behoben werden. Ebenso kann eine mangelhafte Koplanarität oder Orthogonalität der Laser behoben werden. Die Auswertung der Messergebnisse kann dabei in an sich bekannter Weise erfolgen.

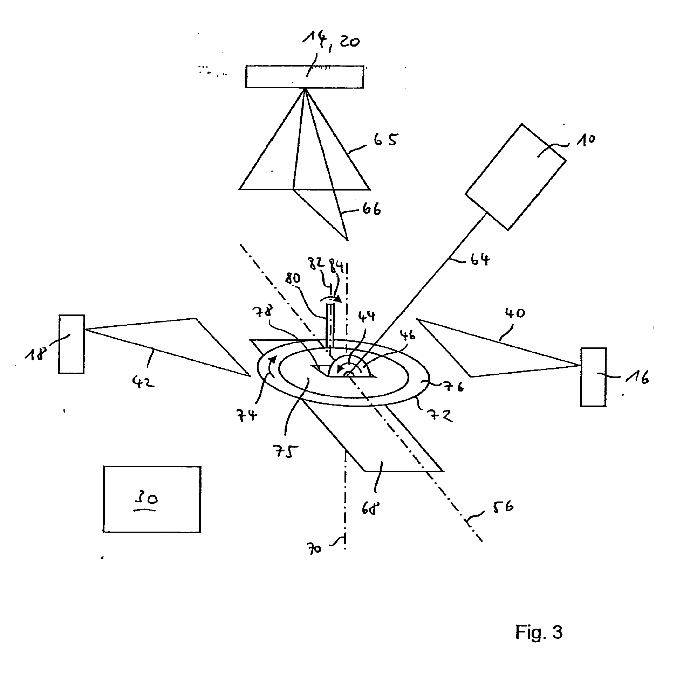

In Figur 3 ist eine erfindungsgemäße Vorrichtung gezeigt. Diese Vorrichtung entspricht weitgehend der in den Figuren 1 und 2 gezeigten Vorrichtung, wobei die Leitungen 28 aus Gründen der Übersichtlichkeit nicht dargestellt sind. Auch sind die in Figur 1 getrennt gezeigten Laser 14, 20 in Figur 3 als kombinierter Laser 14, 20 gezeigt. Neben den von den seitlichen Lasern 16, 18 ausgesandten Strahlungsfeldern 40, 42 sind in Figur 3 auch die beiden in Figur 2 nicht dargestellten Strahlenfelder 65, 66 der oberhalb der Patientenunterlage 68 angeordneten Laser gezeigt. Von der in Figur 2 im Detail gezeigten Vorrichtung zur Ermittlung des Isozentrums des Bestrahlungsgeräts 10 ist in Figur 3 aus Gründen der Übersichtlichkeit nur die Detektorscheibe 46 dargestellt. Dennoch ist die Vorrichtung zur Ermittlung des Isozentrums des Bestrahlungsgeräts 10 in Figur 3 im Übrigen identisch zu der in Figur 2 gezeigten entsprechenden Vorrichtung ausgebildet.

Das Messphantom nach Figur 3 unterscheidet sich von dem in den Figuren 1 und 2 gezeigten Messphantom insbesondere hinsichtlich der Laserdetektoreinrichtung. Anstelle der drei optischen Detektoren 22, 24, 26 ist bei dem Ausführungsbeispiel nach Figur 3 eine Laserdetektoreinrichtung mit einer um eine Rotationsachse 70 drehbaren Detektorscheibe 72 vorgesehen, wie durch den Pfeil 74 veranschaulicht.

Die Detektorscheibe 72 weist eine durch einen ersten Rotationsantrieb (nicht dargestellt) um einen feststehenden zentralen Abschnitt 75 drehbare Ringscheibe 76 auf. Der zentrale Abschnitt 75 besitzt eine etwa rechteckige Ausnehmung 78, in der sich die Detektorscheibe 46 der Vorrichtung zur Ermittlung des Isozentrums des Bestrahlungsgeräts 10 befindet. Während diese Detektorscheibe 46 in einer vertikalen Ebene senkrecht zu der Längsachse der Patientenunterlage 68 angeordnet ist, ist die Detektorscheibe 74 der Laserdetektoreinrichtung in einer waagerechten Ebene parallel zu der Ebene der Oberfläche der Patientenunterlage 68 angeordnet. Von der drehbaren Ringscheibe 76 erstreckt sich in dem Beispiel in Figur 3 nach oben ein Zeilensensor 80. Mittels eines ebenfalls nicht näher dargestellten zweiten Rotationsantriebs ist dieser Sensor 80 um eine in vertikaler Richtung verlaufende Rotationsachse 82 drehbar, wie durch den Pfeil 84 veranschaulicht.

Die Bestimmung des Isozentrums des Bestrahlungsgeräts 10 erfolgt bei der Vorrichtung gemäß Figur 3 in der gleichen Weise wie oben zu Figur 2 erläutert. Für die Ermittlung einer von den Lasern 14, 16, 18, 20 angezeigten Position, insbesondere eines von diesen Lasern angezeigten Isozentrums des Bestrahlungsgeräts 10, wird bei dem Ausführungsbeispiel nach Figur 3 dagegen die Ringscheibe 76 von der Auswerte- und Steuereinrichtung 30 in Rotation versetzt. Während der Drehung der Ringscheibe 76 wird der Sensor 80 ebenfalls in Drehung versetzt derart, dass er während der Drehung der Ringscheibe 76 immer dem Strahlungsfeld eines jeweils zu vermessenden Lasers, beispielsweise dem Bestrahlungsfeld 40 des Lasers 16 zugewandt ist. Die Zeilensensoren des Laserdetektors 80 nehmen dann das Strahlungsfeld 40 auf. Aus der Auswertung der Messergebnisse der Zeilensensoren, insbesondere hinsichtlich der Position der die Laserstrahlung erfassenden Sensoren und den Zeitpunkten der jeweils erfassten Laserstrahlung kann die Position und Neigung der Laserstrahlenfelder, beispielsweise des Strahlenfelds 40 ermittelt werden. Diese Auswertung wird von der Auswerte- und Steuereinrichtung 30 nacheinander für sämtliche der Strahlungsfelder 40, 42, 65, 66 der Laser 14, 16, 18, 20 durchgeführt. Dazu wird die Orientierung des Detektorfeldes des Laserdetektors 80 in geeigneter Weise ausgerichtet. Es ist auf diese Weise möglich, auch die Strahlungsfelder 65, 66 zu vermessen. Allerdings ist bei dem die Sagitallinie erzeugenden Strahlungsfeld 66 eine Messung nur im Bereich zweier Messpositionen möglich, nämlich wenn sich der Laserdetektor 80 auf Höhe der Rotationsachse 56 befindet. Auf Grundlage der Messergebnisse kann wiederum in analoger Weise wie oben zu Figur 2 erläutert durch die Auswerte- und Steuereinrichtung automatisch eine gegebenenfalls erforderliche Neujustage der Laser 14, 16, 18, 20 vorgenommen werden.

## Patentansprüche

1. Vorrichtung zur Ermittlung einer Abweichung zwischen einem durch eine Laseranordnung angezeigten Isozentrum eines Bestrahlungsgeräts (10) und dem tatsächlichen Isozentrum des Bestrahlungsgeräts (10), umfassend
- eine Laseranordnung mit mehreren Lasern (14, 16, 18, 20) und eine Lasersteuereinrichtung (30), die dazu ausgebildet ist, die Laser (14, 16, 18, 20) der Laseranordnung so anzusteuern, dass diese das Isozentrum anzeigen,
- eine Laserdetektoreinrichtung, die dazu ausgebildet ist, von den Lasern (14, 16, 18, 20) ausgesendete Laserstrahlung zu detektieren, und wobei die Laserdetektoreinrichtung weiterhin dazu ausgebildet ist, die Lage und/oder Ausrichtung von durch die Laser (14, 16, 18, 20) ausgesendeten Laserstrahlungsfeldern (40, 42, 65, 66) zu bestimmen, wobei die Laserdetektoreinrichtung mindestens einen eindimensionalen oder zweidimensionalen Laserdetektor umfasst, welcher mindestens ein eindimensionales oder zweidimensionales Sensor-Array umfasst,
- eine Vorrichtung zur Ermittlung des Isozentrums eines Bestrahlungsgeräts (10) zur medizinischen Bestrahlung eines Patienten (12) mit hochenergetischer Strahlung, umfassend eine Strahlendetektoreinrichtung mit einer Detektorscheibe (46), wobei auf mindestens einer Scheibenfläche der Detektorscheibe (46) eine Mehrzahl von Strahlendetektoren (60) zum Detektieren von durch das Bestrahlungsgerät (10) ausgesendeter hochenergetischer Strahlung angeordnet ist, wobei die Strahlendetektoren (60) entlang einer S-Form auf der Scheibenfläche verteilt angeordnet sind, und wobei ein Rotationsantrieb vorgesehen ist, mit dem die Detektorscheibe (46) um ihre Zentralachse drehbar ist, und umfassend eine Auswerteeinrichtung (30), die dazu ausgebildet ist, aus den von den Strahlendetektoren (60) detektierten Messwerten das Isozentrum des Bestrahlungsgeräts (10) zu bestimmen
- eine Auswerteeinrichtung (30), die dazu ausgebildet ist, aus von der Laserdetektoreinrichtung und der Strahlendetektoreinrichtung aufgenommenen Messdaten eine Abweichung zwischen dem durch die Laseranordnung angezeigten Isozentrum und dem tatsächlichen Isozentrum zu ermitteln.

2. Vorrichtung nach Anspruch 1, **dadurch gekennzeichnet, dass** die Steuereinrichtung (30) dazu ausgebildet ist, auf Grundlage der von der Auswerteeinrichtung (30) ermittelten Positionsabweichung die Laser (14, 16, 18, 20) automatisch so anzusteuern, dass der von ihnen angezeigte Schnittpunkt dem tatsächlichen Isozentrum entspricht.

3. Vorrichtung nach einem der vorhergehenden Ansprüche, **dadurch gekennzeichnet, dass** der mindestens eine Laserdetektor mindestens einen Matrixdetektor umfasst.

4. Vorrichtung nach einem der vorhergehenden Ansprüche, **dadurch gekennzeichnet, dass** weiterhin ein Translationsantrieb vorgesehen ist, mit dem die Detektorscheibe (46) entlang ihrer Zentralachse verschiebbar ist.

5. Vorrichtung nach einem der vorhergehenden Ansprüche, **dadurch gekennzeichnet, dass** die beiden Arme der S-Form jeweils einen Radius besitzen, der doppelt so groß ist wie der Radius der Scheibe.

6. Vorrichtung nach einem der vorhergehenden Ansprüche, **dadurch gekennzeichnet, dass** die Vorrichtung weiterhin ein Bestrahlungsgerät (10) zur medizinischen Bestrahlung eines Patienten (12) mit hochenergetischer Strahlung und eine Strahlungsdetektorsteuereinrichtung (30) umfasst, wobei die Strahlungsdetektorsteuereinrichtung (30) dazu ausgebildet ist, das Bestrahlungsgerät (10) so anzusteuern, dass es um seine Rotationsachse gedreht wird und während der Rotation von dem Bestrahlungsgerät (10) mehrfach ein Zentralstrahl (64) ausgesandt wird, und wobei die Strahlungsdetektorsteuereinrichtung (30) weiter dazu ausgebildet ist, während des Aussendens des Zentralstrahls (64) die Deteklorscheibe (46, 72) der Strahlendetektoreinrichtung um ihre Zentralachse zu drehen und den mindestens einen Strahlendetektor (60) so anzusteuern, dass dieser die von dem Bestrahlungsgerät (10) ausgesandten Zentralstrahlen (64) detektiert.

## Claims

1. A device for determining a deviation between an isocentre of an irradiation device (10) displayed by a laser arrangement and the actual isocentre of the irradiation device (10) comprising
- a laser arrangement with a plurality of lasers (14, 16, 18, 20) and a laser control apparatus (30) which is designed to control lasers (14, 16, 18, 20) of the laser arrangement such that they display the isocentre,
- a laser detector apparatus that is designed to detect laser radiation emitted by the lasers (14, 16, 18, 20), and wherein the laser detector apparatus is furthermore designed to determine the position and/or alignment of laser radiation fields (40, 42, 65, 66) emitted by the lasers (14, 16, 18, 20), wherein the laser detector apparatus comprises at least one one-dimensional or two-dimensional laser detector that comprises at least one one-dimensional or two dimensional sensor array,
- a device for determining the isocentre of an irradiation device (10) for medically irradiating a patient (12) with high-energy radiation comprising a beam detection apparatus with a detector disc (46), wherein on at least one disc surface of the detector disc (46), there is disposed a plurality of beam detectors (60) for detecting high-energy radiation emitted by the irradiation device (10), wherein the beam detectors (60) are disposed distributed along an S-shape on the disk surface, and wherein a rotary drive is provided by means of which the detector disc (46) is rotatable about its central axis, and comprising an evaluation device (30) which is designed to determine the isocentre of the irradiation device (10) from the measured values detected by the beam detectors (60)
- an evaluation apparatus (30) which is designed to determine a deviation between the isocentre displayed by the laser arrangement and the actual isocentre from the measured data recorded by the laser detector apparatus and the beam detector apparatus.

2. The device according to claim 1, **characterized in that** the control apparatus (30) is designed to automatically control the lasers (14, 16, 18, 20) on the basis of the positional deviation determined by the evaluation apparatus (30) such that the intersection displayed by the lasers corresponds to the actual isocentre.

3. The device according to any one of the preceding claims, **characterized in that** the at least one laser detector comprises at least one matrix detector.

4. The device according to any one of the preceding claims, **characterized in that** a translation drive is also provided by means of which the detector disc (46) can be moved along its central axis.

5. The device according to any one of the preceding claims, **characterized in that** the two arms of the S-shape each possess a radius that is twice as large as the radius of the disc.

6. The device according to any one of the preceding claims, **characterized in that** the device furthermore comprises an irradiation device (10) for medically irradiating a patient (12) with high-energy radiation and a radiation detector control apparatus (30), wherein the radiation detector control apparatus (30) is designed to control the irradiation device (10) such that it is rotated about its rotary axis and, during the rotation of the irradiation device (10), a central beam (64) is transmitted several times, and wherein the radiation detector control apparatus (30) is furthermore designed to rotate the detector disc (46, 72) of the beam detector apparatus about its central axis while the central beam (64) is being transmitted and to control the at least one beam detector (60) such that the beam detector detects the central beams (64) emitted by the irradiation device (10).

## Revendications

1. Dispositif de détermination d'une déviation entre un isocentre indiqué par un agencement de lasers d'un appareil de rayonnement (10) et l'isocentre effectif de l'appareil de rayonnement (10), comprenant
- un agencement de lasers avec plusieurs lasers (14, 16, 18, 20) et un dispositif de commande de laser (30) qui est réalisé pour commander les lasers (14, 16, 18, 20) de l'agencement de lasers de sorte que ceux-ci indiquent l'isocentre,
- un dispositif de détecteur de laser qui est réalisé pour détecter le rayonnement laser émis par les lasers (14, 16, 18, 20), et dans lequel le dispositif de détecteur de laser est en outre réalisé pour déterminer la position et/ou l'orientation de champs de rayonnement laser (40, 42, 65, 66) émis par les lasers (14, 16, 18, 20), dans lequel le dispositif de détecteur de laser comprend au moins un détecteur de laser unidimensionnel ou bidimensionnel qui comprend au moins un réseau de capteurs unidimensionnel ou bidimensionnel,
- un dispositif de détermination de l'isocentre d'un appareil de rayonnement (10) pour l'irradiation médicale d'un patient (12) avec un rayonnement à haute énergie, comprenant un dispositif de détecteur de rayons avec un disque de détecteur (46), dans lequel une pluralité de détecteurs de rayons (60) est disposée sur au moins une face de disque du disque de détecteur (46) pour la détection d'un rayonnement à haute énergie émis par l'appareil de rayonnement (10), dans lequel les détecteurs de rayons (60) sont disposés de manière répartie le long d'une forme de S sur la face de disque, et dans lequel un entraînement à rotation est prévu avec lequel le disque de détecteur (46) peut tourner autour de son axe central, et comprenant un dispositif d'évaluation (30) qui est réalisé pour déterminer l'isocentre de l'appareil de rayonnement (10) à partir des valeurs de mesure détectées par les détecteurs de rayons (60),
- un dispositif d'évaluation (30) qui est réalisé pour déterminer une déviation entre l'isocentre indiqué par l'agencement de lasers et l'isocentre effectif à partir des données de mesure enregistrées par le dispositif de détecteur de laser et le dispositif de détecteur de rayons.

2. Dispositif selon la revendication 1, **caractérisé en ce que** le dispositif de commande (30) est réalisé pour commander automatiquement les lasers (14, 16, 18, 20) sur la base de la déviation de position déterminée par le dispositif d'évaluation (30) de sorte que l'intersection indiquée par eux correspond à l'isocentre effectif.

3. Dispositif selon une des revendications précédentes, **caractérisé en ce que** l'au moins un détecteur de laser comprend au moins un détecteur de matrice.

4. Dispositif selon une des revendications précédentes, **caractérisé en ce qu'**un entraînement à translation est en outre prévu avec lequel le disque de détecteur (46) peut coulisser le long de son axe central.

5. Dispositif selon une des revendications précédentes, **caractérisé en ce que** les deux bras de la forme de S possèdent chacun un rayon qui est le double du rayon du disque.

6. Dispositif selon une des revendications précédentes, **caractérisé en ce que** le dispositif comprend en outre un appareil de rayonnement (10) pour l'irradiation médicale d'un patient (12) avec un rayonnement à haute énergie et un dispositif de commande de détecteur de rayonnement (30), dans lequel le dispositif de commande de détecteur de rayonnement (30) est réalisé pour commander l'appareil de rayonnement (10) de sorte qu'il est tourné autour de son axe de rotation et qu'un rayon central (64) est émis plusieurs fois par l'appareil de rayonnement (10) pendant la rotation, et dans lequel le dispositif de commande de détecteur de rayonnement (30) est en outre réalisé pour faire tourner le disque de détecteur (46, 72) du dispositif de détecteur de rayons autour de son axe central pendant l'émission du rayon central (64) et commander l'au moins un détecteur de rayons (60) de sorte que celui-ci détecte les rayons centraux (64) émis par l'appareil de rayonnement (10).
